(19) Europäisches Patentamt
European Patent Office
Office européen des brevets

(11) Veröffentlichungsnummer: **0 215 120**
**B1**

# (12) EUROPÄISCHE PATENTSCHRIFT

(45) Veröffentlichungstag der Patentschrift: **23.08.89**

(51) Int. Cl.⁴: **C 07 C 121/60**, C 07 C 121/75, C 07 C 13/28, C 07 C 25/18, C 07 C 121/46, C 07 C 121/48, C 07 C 43/21, C 07 C 43/184

(21) Anmeldenummer: **86902808.4**

(22) Anmeldetag: **21.03.86**

(86) Internationale Anmeldenummer: **PCT/EP 86/00164**

(87) Internationale Veröffentlichungsnummer: **WO 86/05486 (25.09.86 Gazette 86/21)**

## (54) CYCLOHEXANDERIVATE.

(30) Priorität: **22.03.85 DE 3510432**

(43) Veröffentlichungstag der Anmeldung: **25.03.87 Patentblatt 87/13**

(45) Bekanntmachung des Hinweises auf die Patenterteilung: **23.08.89 Patentblatt 89/34**

(84) Benannte Vertragsstaaten: **CH DE FR GB LI NL**

(56) Entgegenhaltungen:
**EP-A-0 063 003**
**DE-A-3 221 941**

(73) Patentinhaber: **MERCK PATENT GESELLSCHAFT MIT BESCHRÄNKTER HAFTUNG, Frankfurter Strasse 250 Postfach 4119, D-6100 Darmstadt (DE)**

(72) Erfinder: **POHL, Ludwig, Niebergallweg 5, D-6100 Darmstadt (DE)**
Erfinder: **SCHEUBLE, Bernhard, Am Grenzweg 18, D-6146 Alsbach (DE)**
Erfinder: **HITTICH, Reinhard, Am Kirchberg 11, D-6101 Modautal 1 (DE)**
Erfinder: **EIDENSCHINK, Rudolf, Kornblumenstrasse 1, D-6115 Münster (DE)**
Erfinder: **KURMEIER, Hans- Adolf, Hinter der Schule 3a, D-6104 Seeheim- Jugenheim (DE)**
Erfinder: **WÄCHTLER, Andreas, Goethestrasse 34, D-6103 Griesheim (DE)**

**EP 0 215 120 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft Cyclohexanderivate der Formel I

$R^1$-$A^1$-$Z^1$-$A^2$-$R^2$ I

worin

| | |
|---|---|
| $R^1$ und $R^2$ | jeweils eine Alkylgruppe mit 1 - 10 C-Atomen, worin auch eine oder zwei nicht benachbarte $CH_2$-Gruppen durch O-Atome und/oder -CO-Gruppen und/oder -CO-O-Gruppen und/oder -CH=CH-Gruppen ersetzt sein können, einer der Reste $R^1$ und $R^2$ auch H, F, Cl, Br, CN oder $R^3$-$A^3$-$Z^2$-, |
| $A^1$ | -A-, $A^4$ -A- oder -A-$A^4$-, |
| A | eine in 2-, 3-, 5- und/oder 6-Stellung ein- oder mehrfach durch F und/oder Cl und/oder Br und/oder CN und/oder eine Alkylgruppe oder eine fluorierte Alkylgruppe mit jeweils 1 - 10 C-Atomen, worin auch eine oder zwei nicht benachbarte $CH_2$-Gruppen durch O-Atome und/oder -CO-Gruppen und/oder -CO-O-Gruppen ersetzt sein können, substituierte trans-1,4-Cyclohexylengruppe, die gegebenenfalls auch in 1- und/oder 4-Stellung substituiert sein kann, |
| $A^2$, $A^3$ und $A^4$ | jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome und/ oder NO ersetzt sein können, 1,4-Cyclohexylen, worin auch ein oder zwei nicht benachbarte $CH_2$-Gruppen durch O-Atome ersetzt sein können, 1,3-Dithian-2,5-diyl, Piperidin-1,4-diyl, 1,4-Bicyclo(2,2,2)-octylen-, Decahydronaphthalin-2,6-diyl- oder 1,2,3,4-Tetrahydronaphthalin-2,6-diyl-Gruppen, |
| $Z^1$ und $Z^2$ | jeweils -CO-O-, -O-CO-, $-CH_2CH_2-$, $CH_2$-, $-CH_2O$ oder eine Einfachbindung, und |
| $R^3$ | H, eine Alkylgruppe mit 1 - 10, worin auch eine oder zwei nicht benachbarte $CH_2$-Gruppen durch O-Atome und/oder -CO-Gruppen und/oder -CO-O-Gruppen und/oder -CH=CH-Gruppen ersetzt sein können, F, Cl, Br oder CN bedeutet. |

mit der Maßgabe, daß im Falle $Z^1$ = -CO-O- $A^1$ in β-Position zur -CO-O-Brücke keinen äquatorialen Substituenten trägt.

Der Einfachheit halber bedeuten im folgenden Phe eine 1,4-Phenylengruppe, Cy eine 1,4-Cyclohexylengruppe, Dio eine 1,3-Dioxan-2,5-diylgruppe, Bi eine Bicyclo-(2,2,2)-octylengruppe, Pip eine Piperidin-1,4-diylgruppe, Pyr eine Pyrimidin-2,5-diylgruppe, Pyn eine Pyridazin-3,6-diyl Gruppe, die gegebenenfalls auch als N-Oxid vorliegen kann, Dit eine 1,3-Dithian-2,5-diylgruppe und Dec eine Decahydronaphthalin-2,6-diylgruppe.

Ähnliche Verbindungen sind z. B. aus der DE-PS-2 636 684 bekannt. Die dort angegebenen Verbindungen enthalten jedoch im Gegensatz zu den vorliegenden keine trisubstituierten Cyclohexanringe. Derivate der trans-4-substituierten r-2-Methylcyclohexancarbonsäure sind aus EP-OS-0 063 003 bekannt. Es zeigte sich jedoch, daß die erfindungsgemäßen Derivate der 2-Methylcyclohexancarbonsäure mit axialer Methylgruppe in β-Position zur -CO-O-Brücke eine günstigere 'rod-like'-Struktur aufweisen, da die axiale Methylgruppe sich nahezu vollständig in Rotationswinkel des Cyclohexanringes befindet.

Die Verbindungen der Formel I können wie ähnliche Verbindungen als Komponenten flüssigkristalliner Dielektrika verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen mit sehr kleiner optischer Anisotropie und vergleichsweise niedriger Viskosität herstellbar.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken und/oder störende smektische Phasenbereiche zu unterdrücken. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt, oder daß man zur Herstellung von Estern der Formel I eine entsprechende

Carbonsäure oder eines ihrer reaktionsentsprechenden Derivate mit einem entsprechenden Alkohol oder einem seiner reaktionsfähigen Derivate umsetzt,

oder daß man zur Herstellung von Dioxanderivaten der Formel I einen entsprechenden Aldehyd mit einem entsprechenden Diol umsetzt,

oder daß man zur Herstellung von Nitrilen der Formel I ein entsprechendes Carbonsäureamid dehydratisiert oder ein entsprechendes Carbonsäurehalogenid mit Sulfamid umsetzt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Vor- und nachstehend haben $R^1$, $R^2$, $R^3$, $A^1$, $A^2$, $A^3$, $A^4$, A, $Z^1$ und $Z^2$ die angegebende Bedeutung, sofern nicht ausdrücklich etwas anderes vermerkt ist.

Die Verbindungen der Formel I umfassen dementsprechend insbesondere Verbindungen der Teilformeln Ia und Ib (mit zwei Ringen)

| | |
|---|---|
| $R^1$-A-$A^2$-$R^2$ | Ia |
| $R^1$-A-$Z^1$-$A^2$-$R^2$ | Ib |

Ic bis Ii (mit drei Ringen),

| | |
|---|---|
| $R^1$-$A^4$-A-$A^2$-$R^2$ | Ic |
| $R^1$-A-$A^4$-$A^2$-$R^2$ | Id |
| $R^1$-$A^4$-A-$Z^1$-$A^2$-$R^2$ | Ie |
| $R^1$-A-$A^4$-$Z^1$-$A^2$-$R^2$ | If |
| $R^1$-A-$Z^1$-$A^2$-$A^3$-$R^3$ | Ig |
| $R^3$-$A^3$-$Z^2$-A-$Z^1$-$A^2$-$R^2$ | Ih |
| $R^1$-A-$Z^1$-$A^2$-$Z^2$-$A^3$-$R^3$ | Ii |

sowie Ij bis It (mit vier Ringen)

| | |
|---|---|
| $R^1$-$A^4$-A-$A^2$-$A^3$-$R^3$ | Ij |
| $R^1$-A-$A^4$-$A^2$-$A^3$-$R^3$ | Ik |
| $R^3$-$A^3$-$Z^2$-$A^4$-A-$A^2$-$R^2$ | Il |
| $R^3$-$A^3$-$A^4$-A-$Z^1$-$A^2$-$R^2$ | Im |
| $R^1$-A-$A^4$-$A^2$-$Z^2$-$A^3$-$R^3$ | In |
| $R^1$-A-$A^4$-$Z^1$-$A^2$-$A^3$-$R^3$ | Io |
| $R^1$-$A^4$-A-$Z^1$-$A^2$-$A^3$-$R^3$ | Ip |
| $R^1$-$A^4$-A-$Z^1$-$A^2$-$Z^2$-$A^3$-$R^3$ | Iq |
| $R^1$-A-$A^4$-$Z^1$-$A^2$-$Z^2$-$A^3$-$R^3$ | Ir |
| $R^3$-$A^3$-$Z^2$-$A^4$-A-$Z^1$-$A^2$-$R^2$ | Is |
| $R^3$-$A^3$-$Z^2$-A-$A^4$-$Z^1$-$A^2$-$R^2$ | It |

Darunter sind diejenigen der Formeln Ia, Ib, Ic, Id, Ie, If, IG, Ij und Ik besonders bevorzugt.

Die bevorzugten Verbindungen der Formel Ia umfassen solche der Teilformeln Ia1 und Ia3:

| | |
|---|---|
| $R^1$-A-Ph-$R^2$ | Ia1 |
| $R^1$-A-Cy-$R^2$ | Ia2 |
| $R^1$-A-Bi-$R^2$ | Ia3 |

Darunter sind diejenigen der Teilformeln Ia1 und Ia2 besonders bevorzugt.

Die bevorzugten Verbindungen der Formel Ib umfassen solche der Teilformeln Ib1 bis Ib3:

| | |
|---|---|
| $R^1$-A-$Z^1$-Ph-$R^2$ | Ib1 |
| $R^1$-A-$Z^1$-Cy-$R^2$ | Ib2 |
| $R^1$-A-$Z^1$-Bi-$R^2$ | Ib3 |

Darunter sind diejenigen der Teilformeln Ib1 und Ib2, insbesondere diejenigen worin $Z^1$ -CO-O-, O-CO- oder $-CH_2CH_2-$ bedeutet, besonders bevorzugt.

Die bevorzugten Verbindungen der Formel Ic umfassen solche der Teilformeln Ic1 und Ic2:

| | |
|---|---|
| $R^1$-Cy-A-Cy-$R^2$ | Ic1 |
| $R^1$-Cy-A-Ph-$R^2$ | Ic2 |

Die bevorzugten Verbindungen der Formel Id umfassen solche der Teilformeln Id1 bis Id4:

$R^1$-A-Cy-Cy-$R^2$ Id1
$R^1$-A-Ph-Ph-$R^2$ Id2
$R^1$-A-Ph-Cy-$R^2$ Id3
$R^1$-A-Cy-Ph-$R^2$ Id4

Die bevorzugten Verbindungen der Formel Ie umfassen solche der Teilformeln Ie1 bis Ie3:

$R^1$-Cy-A-$Z^1$-Cy-$R^2$ Ie1
$R^1$-Cy-A-$Z^1$-Ph-$R^2$ Ie2
$R^1$-Ph-A-$Z^1$-Cy-$R^2$ Ie3

Darunter sind diejenigen der Teilformeln Ie1, insbesondere diejenigen worin $Z^1$ -CO-O-, -O-CO- oder -$CH_2CH_2$- bedeutet, besonders bevorzugt.

Die bevorzugten Verbindungen der Formel If umfassen solche der Teilformeln If1 bis If4:

$R^1$-A-Cy-$Z^1$-Cy-$R^2$ If1
$R^1$-A-Ph-$Z^1$-Ph-$R^2$ If2
$R^1$-A-Ph-$Z^1$-Cy-$R^2$ If3
$R^1$-A-Cy-$Z^1$-Ph-$R^2$ If4

Darunter sind diejenigen der Teilformeln If1, If2 und If3, insbesondere diejenigen worin $Z^1$ -CO-O-, -O-CO- oder -$CH_2CH_2$-, insbesondere -CO-O-, bedeutet, besonders bevorzugt.

Die bevorzugten-Verbindungen der Formel Ig umfassen solche der Teilformeln Ig1 und Ig3:

$R^1$-A-$Z^1$-Cy-Cy-$R^3$ Ig1
$R^1$-A-$Z^1$-Ph-Cy-$R^2$ Ig2
$R^1$-A-$Z^1$-Ph-Ph-$R^2$ Ig3

Darunter sind diejenigen besonders bevorzugt, worin $Z^1$ -O-CO-, -CO-O- oder -$CH_2CH_2$- bedeutet.

Die bevorzugten Verbindungen der Formel Ij umfassen solche der Teilformeln Ij1 und Ij2:

$R^1$-Cy-A-Ph-Ph-$R^3$ Ij1
$R^1$-Cy-A-Ph-Cy-$R^3$ Ij2

Die bevorzugten Verbindungen der Formel Ik umfassen solche der Teilformeln Ik1 und Ik2:

$R^1$-A-Ph-Ph-Cy-$R^3$ Ik1
$R^1$-A-Ph-Cy-Cy-$R^3$ Ik2

In den Verbindungen der vor- und nachstehenden Formeln bedeuten $R^1$, $R^2$ bzw. $R^3$ vorzugsweise Alkyl, ferner Alkoxy- oder eine andere Oxaalkylgruppe.

Weiterhin bevorzugt sind Verbindungen der vor- und nachstehenden Formeln worin einer der Rest $R^1$, $R^2$ bzw. $R^3$ -CO-Alkyl, -O-CO-Alkyl, -CO-O-Alkyl oder CN und der andere Alkyl bedeutet.

In den bevorzugten Verbindungen der vor- und nachstehenden Formeln können die Alkylreste, in denen auch eine $CH_2$-Gruppe (Alkoxy bzw. Oxaalkyl) durch ein O-Atom ersetzt sein kann, geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= 2-Methoxymethyl), 2- (= Ethoxymethyl) oder 3-Oxabutyl (= 2-Methoxypentyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Methoxy, Octoxy oder Nonoxy.

$A^2$, $A^3$ und $A^4$ sind bevorzugt Cy oder PH. $Z^1$ und $Z^2$ sind bevorzugt Einfachbindungen, in zweiter Linie bevorzugt -O-CO-, -CO-O- oder -$CH_2CH_2$-Gruppen.

A ist bevorzugt eine Gruppe ausgewählt aus den Formeln (A) bis (G),

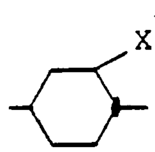
(A)

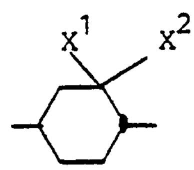
(B)

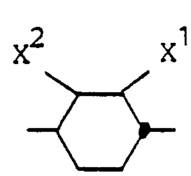
(C)

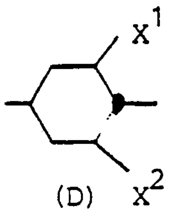
(D)

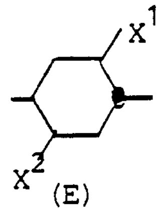
(E)

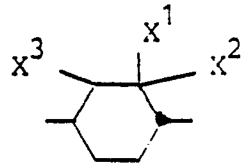
(F)

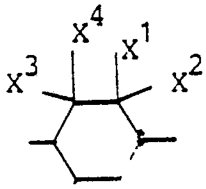
(G)

worin $X^1$, $X^2$, $X^3$ und $X^4$ jeweils unabhängig voneinander F, Cl, Br, CN, Alkyl, Alkoxy, Oxaalkyl, Alkanoyl, Alkanoxyloxy oder Alkoxycarbonyl mit jeweils 1 bis 10 C-Atomen bedeuten.

A umfaßt auch die Spiegelbilder der Formeln (A) bis (G). Die Gruppen der Formeln (A) bis (G) können in 1- oder 4-Stellung einen zusätzlichen axialen Substituenten Q tragen. Q ist vorzugsweise F, CN oder $CH_3$. Besonders bevorzugte ist CN.

Bevorzugte Bedeutungen von $X^1$, $X^2$, $X^3$ und $X^4$ sind F, Cl, CN, $-CH_3$, $-CH_2CH_3$ und $-OCH_3$.

Besonders bevorzugt sind die Gruppen F, CN und $CH_3$, insbesondere CN und $CH_3$.

Verbindungen der vor- und nachstehenden Formeln mit verzweigter Flügelgruppen $R^1$, $R^2$ bzw. $R^3$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Solche Verbindungen sind als Komponenten smektischer Mischungen mit ferroelektrischen Eigenschaften verwendbar.

Bevorzugte verzweigte Reste sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhetoxy, 1-Methylheptoxy, 2-Oxa-3-methylbutyl, 3-Oxa-4-methylpentyl.

Unter den Verbindungen der Formel I sowie Ia bis It sind diejenigen bevorzugt, in denen mindestens einer der darin enthaltenen Reste eine der angegebenen bevorzugten Bedeutungen hat.

Besonders bevorzugt sind Verbindungen der Formel I enthaltend das Strukturelement

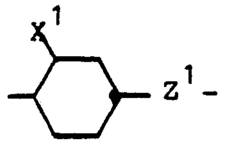 ,

worin der Cyclohexanring ggf. auch in 1- oder 4-Stellung substituiert sein kann. $X^1$ ist vorzugsweise $CH_3$.

In den Verbindungen der vorstehenden genannten Formeln sind diejenigen Stereoisomeren bevorzugt, in den die Substituenten $R^1$-, $R^1$-$A^4$-, $R^2$-$A^2$-$Z^1$- bzw. $R^2$-$A^2$-$Z^1$-$A^4$- in 1- und 4-Position des Ringes A trans-ständig sind und die äquatoriale Stellung einnehmen, während der gegebenenfalls vorhandene zusätzliche Substituent Q an A in 1- oder 4-Position eine axiale Stellung einnimmt. Diese sind in der Regel stabiler; in vielen Fällen lassen sich die cis-Verbindungen (oder Gemische) durch Behandeln mit einer Base, z. B. mit K-tert.-Butylat in einem inerten Lösungsmittel wie Dimethylsulfoxid, in die trans-Verbindungen umwandeln.

Die Substituenten $X^1$, $X^2$, $X^3$ und $X^4$ in den Gruppen der Formeln (A), (C), (D), (E) und (F) können äquatoriale oder axiale Stellungen einnehmen. Vorzugsweise sind diese Substiuenten in äquatorialen Positionen.

Diejenigen der vorstehend genannten Formeln, die eine oder mehrere Gruppen Dio, Dit, Pip und/oder Pyr enthalten, umschließen jeweils die beiden möglichen 2,5-(Dio, Dit, Pyr) bzw. 1,4-Stellungsisomeren (Pip).

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z. B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer $-CH_2CH_2-$Gruppe eine $-CH=CH-$Gruppe und/oder an Stelle einer $-CH_2-$Gruppe eine -CO-Gruppe und/oder an Stelle eines H-Atoms eine freie oder eine funktionell (z. B. in Form ihres p-Toluolsulfonats) abgewandelte OH-Gruppen enthalten.

Die Reduktion kann z. B. erfolgen durch katalytische Hydrierung bei Temperaturen zwischen etwa 0° und etwa 200° sowie Drucken zwischen etwa 1 und 200 bar in einem inerten Lösungsmittel, z. B. einem Alkohol wie Methanol, Ethanol oder Isopropanol, einem Ester wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z. B. $PtO_2$, $PdO_2$), auf einem Träger (z. B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder $-CH_2CH_2-$Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyoxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden; so entstehen z. B. aus 1-Cyancyclohexenderivaten die entsprechenden Cyclohexanderivate.

Verbindungen der Formel I sind weiterhin erhältlich, indem man an ein entsprechendes Cyclohexenderivat (das Formel I entspricht, aber an Stelle des Restes A eine 1-Cyclohexen-1,4-diylgruppe enthält, die 1 oder 2 weitere F-, Cl- oder Br-Atome und/oder CN-Gruppen tragen kann) eine Verbindung der Formel $Q^1Q^2$ (z. B. HBr, BrCN, BrF, BrCl, $Br_2$) radikalisch anlagert.

Diese Anlagerung gelingt z. B. in Gegenwart eines inerten Lösungsmittels, z. B. eines halogenierten Kohlenwasserstoffs wie $CH_2Cl_2$ oder $CHCl_3$ bei Temperaturen zwischen etwa -10 und +150° und Drucken zwischen etwa 1 und 100 bar. Ein Zusatz von Radikalininitiatoren oder die Durchführung als Photoreaktion kann günstig sein.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischem Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedinungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton

oder DMF versetzt, zweckmäßig bei Temperaturen zwischen etwa -25° und +20°.

Dioxanderivate bzw. Dithianderivate der Formel I werden zweckmäßig durch Reaktion eines entsprechenden Aldehyds (oder eines seiner reaktionsfähigen Derivate) mit einem entsprechenden 1,3-Diol bzw. einem entsprechenden 1,3-Dithiol (oder einem ihrer reaktionsfähigen Derivate) hergestellt, vorzugsweise in Gegenwart eines inerten Lösungsmittels wie Benzol oder Toluol und/oder eines Katalysators, z. B. einer starken Säure wie Schwefelsäure, Benzol- oder p-Toluolsulfonsäure, bei Temperaturen zwischen 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, alle können ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Zur Herstellung von Nitrilen der Formel I können entsprechende Säureamide, z. B. solche in denen an Stelle des Restes X eine $CONH_2$-Gruppe steht, dehydratisiert werden. Die Amide sind z. B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z. B. als Doppelverbindungen mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z. B. Basen wie Pyridin oder Triethylamin, aromatische Kohlenwasserstoffe wie Benzol, Toluol, oder Xylol oder Amide wie DMF i-n Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z. B. durch Behandeln mit NaH, $HaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Fluorverbindungen der Formel I, worin A eine durch F substituierte 1,4-Cyclohexylengruppe bedeutet, die zusätzlich weitere Substituenten tragen kann, sind durch Behandeln der entsprechenden Hydroxyverbindungen oder Brom- oder Chlorverbindungen mit einem Fluorierungsmittel erhältlich. Als Fluorierungsmittel können alle für diese Austauschreaktionen bekannten Verbindungen verwendet werden, beispielsweise Diethylaminschwefeltrifluorid (J. Org. Chem. 40 (5), 574 - 8 (1975). Die Hydroxy-, Brom- und Chlor-Verbindungen sind beispielsweise aus den entsprechenden Cyclohexenverbindungen durch Anlagerung von $H_2O$, HBr oder HCl erhältlich.

Zur Herstellung von Nitrilen der Formel I können auch entsprechende Chlor- oder Bromverbindungen der Formel I mit einem Cyanid umgesetzt werden, zweckmäßig mit einem Metallcyanid wie NaCN, KCN oder $Cu_2(CN)_2$, z. B. in Gegenwart von Pyridin in einem inerten Lösungsmittel wie DMF oder N-Methylpyrrolidon bei Temperaturen zwischen 20° und 200°.

Die erfindungsgemäßen flüssigkristallinen Phasen bestehen aus 2 bis 20, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel I' charakterisieren,

R'-L-G-E-R''  I'

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | -COO-Phe-COO- |

oder eine C-C-Einfachbindung,

Y Halogen, vorzugsweise Chlor, oder -CN, und

R' und R'' Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95 %, einer oder mehrerer Verbindungen der Formel I. Weiterhin bevorzugt sind erfindungsgemäße flüssigkristalline Phasen, enthaltend 0,1 - 40, vorzugsweise 0,5 - 30 % einer oder mehrerer Verbindungen der Formel I.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammoniumtetraphenylboranat oder Komplexsalze von Kronenethern (vgl. z. B. I. Haller et al., Mol. Cryst. Liq. Cryst. Band 24, Seiten 249 - 258 (1973)) zur Verbesserung der Leitfähigkeit, pleochroitische Farbstoffe zur Herstellung farbiger Guest-Host-Systeme oder Substanzen zur Veränderung der dielektrischen Anisotrophie, der Viskosität und/oder der Orientierung der nematischen Phasen zugesetzt werden. Derartige Substazen sind z. B. in den DE-OS-2 209 127, 2 240 864, 2 321 632, 2 338 281, 2 450 088, 2 637 430, 2 853 728 und 2 902 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

**Beispiel 1**

Eine Lösung von 56 g 2-Methyl-1-(p-n-pentylphenyl)-4-n-propylcyclohexen [erhältlich durch Umsetzung von 2-Methyl-4-propylcyclohexanon (erhältlich durch Umsetzung von 4-Propylcyclohexanon mit NaH und Kohlensäurediethylester, Methylierung des erhaltenen Cyclohexancarbonsäureesters mit Methyljodid/NaOEt/EtOH, Verseifung des Esters und Decarboxylierung) mit der aus p-n-Pentylbrombenzol erhaltenen Grignardverbindung, nachfolgender Hydrolyse und Wasserabspaltung mit p-Toluolsulfonsäure/Toluol] in 500 ml THF wird an 8 g 10 %-igen Pd/C bei 40° und 1 bar bis zur Aufnahme von 0,2 Mol $H_2$ hydriert. Man filtriert, dampft ein und trennt das erhaltene Isomerengemisch durch Chromatographie und Kristallisation. Man erhält r-2-Methyl-t-1-(p-n-pentylphenyl)-c-4-n-propylcyclohexan und r-2-Methyl-c-1-(p-n-pentylphenyl)-t-4-n-propylcyclohexan.

Analog werden hergestellt:

2-Methyl-1-(p-butylphenyl)-4-propylcyclohexan
2-Methyl-1-(p-propylphenyl)-4-propylcyclohexan
2-Methyl-1-(p-ethylphenyl)-4-propylcyclohexan
2-Methyl-1-(p-methoxyphenyl)-4-propylcyclohexan
2-Methyl-1-(p-ethoxyphenyl)-4-propylcyclohexan
2-Methyl-1-(p-propoxyphenyl)-4-propylcyclohexan
2-Methyl-1-(p-cyanphenyl)-4-propylcyclohexan

2-Methyl-1-(p-heptylphenyl)-4-pentylcyclohexan
2-Methyl-1-(p-pentylphenyl)-4-pentylcyclohexan
2-Methyl-1-(p-butylphenyl)-4-pentylcyclohexan
2-Methyl-1-(p-propylphenyl)-4-pentylcyclohexan
2-Methyl-1-(p-ethylphenyl)-4-pentylcyclohexan
2-Methyl-1-(p-methoxyphenyl)-4-pentylcyclohexan
2-Methyl-1-(p-ethoxyphenyl)-4-pentylcyclohexan

2-Methyl-1-(p-propoxyphenyl)-4-pentylcyclohexan
2-Methyl-1-(p-cyanphenyl)-4-pentylcyclohexan

3-Methyl-1-(p-heptylphenyl)-4-butylcyclohexan
3-Methyl-1-(p-pentylphenyl)-4-butylcyclohexan
3-Methyl-1-(p-butylphenyl)-4-butylcyclohexan
3-Methyl-1-(p-propylphenyl)-4-butylcyclohexan
3-Methyl-1-(p-ethylphenyl)-4-butylcyclohexan
3-Methyl-1-(p-methoxyphenyl)-4-butylcyclohexan
3-Methyl-1-(p-ethoxyphenyl)-4-butylcyclohexan
3-Methyl-1-(p-propoxyphenyl)-4-butylcyclohexan
3-Methyl-1-(p-cyanphenyl)-4-butylcyclohexan

**Beispiel 2**

Eine Lösung von 72 g 2-Methyl-1-(p-n-pentylphenyl)-4-(trans-4-n-propylcyclohexyl)-cyclohexen [erhältlich durch Umsetzung von 2-Methyl-4-(trans-4-n-propylcyclohexyl)-cyclohexanon mit NaH und Kohlensäurediethylester, Methylierung des erhaltenen Cyclohexancarbonsäureesters mit Methyljodid/NaOEt/EtOH, Verseifung des Esters und Decarboxylierung) mit der aus p-n-Pentylbrombenzol erhaltenen Grignardverbindung, nachfolgender Hydrolyse und Wasserabspaltung] in 500 ml THF wird an 8 g 10 %-igem Pd/C bei 40° und 1 bar bis zur Aufnahme von 0,2 Mol $H_2$ hydriert. Man filtriert, dampft ein und trennt das erhaltene Isomerengemisch durch Chromatographie und Kristallisation. Man erhält r-2-Methyl-t-1-(p-n-pentylphenyl)-c-4-(trans-4-n-propylcyclohexyl)-cyclohexan und r-2-Methyl-c-1-(p-n-pentylphenyl)-t-4-(trans-4-n-propylcyclohexyl)-cyclohexan.
Analog werden hergestellt:

2-Methyl-1-(p-butylphenyl)-4-(trans-4-propylcyclohexyl)-cyclohexan
2-Methyl-1-(p-propylphenyl)-4-(trans-4-propylcyclohexyl)-cyclohexan
2-Methyl-1-(p-ethylphenyl)-4-(trans-4-propylcyclohexyl)-cyclohexan
2-Methyl-1-(p-methoxyphenyl)-4-(trans-4-propylcyclohexyl)-cyclohexan
2-Methyl-1-(p-ethoxyphenyl)-4-(trans-4-propylcyclohexyl)-cyclohexan
2-Methyl-1-(p-propoxyphenyl)-4-(trans-4-propylcyclohexyl)-cyclohexan
2-Methyl-1-(p-cyanphenyl)-4-(trans-4-propylcyclohexyl)-cyclohexan

2-Methyl-1-(p-propylphenyl)-4-(trans-4-butylcyclohexyl)-cyclohexan
2-Methyl-1-(p-butylphenyl)-4-(trans-4-butylcyclohexyl)-cyclohexan
2-Methyl-1-(p-pentylphenyl)-4-(trans-4-butylcyclohexyl)-cyclohexan
2-Methyl-1-(p-methoxyphenyl)-4-(trans-4-butylcyclohexyl)-cyclohexan
2-Methyl-1-(p-ethoxyphenyl)-4-(trans-4-butylcyclohexyl)-cyclohexan
2-Methyl-1-(p-propoxyphenyl)-4-(trans-4-butylcyclohexyl)-cyclohexan
2-Methyl-1-(p-butoxyphenyl)-4-(trans-4-butylcyclohexyl)-cyclohexan
2-Methyl-1-(p-cyanphenyl)-4-(trans-4-butylcyclohexyl)-cyclohexan

2-Methyl-1-(p-ethylphenyl)-4-(trans-4-pentylcyclohexyl)-cyclohexan
2-Methyl-1-(p-propylphenyl)-4-(trans-4-pentylcyclohexyl)-cyclohexan
2-Methyl-1-(p-butylphenyl)-4-(trans-4-pentylcyclohexyl)-cyclohexan
2-Methyl-1-(p-pentylphenyl)-4-(trans-4-pentylcyclohexyl)-cyclohexan
2-Methyl-1-(p-methoxyphenyl)-4-(trans-4-pentylcyclohexyl)-cyclohexan
2-Methyl-1-(p-ethoxyphenyl)-4-(trans-4-pentylcyclohexyl)-cyclohexan
2-Methyl-1-(p-propoxyphenyl)-4-(trans-4-pentylcyclohexyl)-cyclohexan
2-Methyl-1-(p-butoxyphenyl)-4-(trans-4-pentylcyclohexyl)-cyclohexan
2-Methyl-1-(p-cyanphenyl)-4-(trans-4-pentylcyclohexyl)-cyclohexan

**Beispiel 3**

Ein Gemisch aus 23,8 g 4-(trans-4-n-Propylcyclohexyl)-2-methylcyclohexanol (erhältlich durch Reduktion des entsprechenden Ketons (Beispiel 2) mit Lithiumaluminiumhydrid], 2,4 g NaH und 150 ml Dimethoxyethan wird unter $N_2$-Atmosphäre auf etwa 60°C erhitzt und dann unter kräftigem Rühren 16 g Diethylsulfat zugegeben. Man rührt 12 Stunden bei der angegebenen Temperatur und hydrolysiert dann das abgekühlte Reaktionsgemisch mit wäßrigem THF. Nach üblicher Aufarbeitung erhält man r-2-Methyl-t-1-ethoxy-c-4-(trans-4-n-propylcyclohexyl)-cyclohexan und r-2-Methyl-c-1-ethoxy-t-4-(trans-4-n-propylcyclohexyl)-cyclohexan.
Analog werden hergestellt:

2-Methyl-1-methoxy-4-(trans-4-propylcyclohexyl)-cyclohexan
2-Methyl-1-propoxy-4-(trans-4-propylcyclohexyl)-cyclohexan
2-Methyl-1-butoxy-4-(trans-4-propylcyclohexyl)-cyclohexan

2-Methyl-1-methoxy-4-(trans-4-butylcyclohexyl)-cyclohexan
2-Methyl-1-ethoxy-4-(trans-4-butylcyclohexyl)-cyclohexan
2-Methyl-1-propoxy-4-(trans-4-butylcyclohexyl)-cyclohexan
2-Methyl-1-butoxy-4-(trans-4-butylcyclohexyl)-cyclohexan

2-Methyl-1-methoxy-4-(trans-4-pentylcyclohexyl)-cyclohexan
2-Methyl-1-ethoxy-4-(trans-4-pentylcyclohexyl)-cyclohexan
2-Methyl-1-propoxy-4-(trans-4-pentylcyclohexyl)-cyclohexan
2-Methyl-1-butoxy-4-(trans-4-pentylcyclohexyl)-cyclohexan

2-Ethyl-1-methoxy-4-(trans-4-pentylcyclohexyl)-cyclohexan
2-Ethyl-1-ethoxy-4-(trans-4-pentylcyclohexyl)-cyclohexan
2-Ethyl-1-propoxy-4-(trans-4-pentylcyclohexyl)-cyclohexan
2-Ethyl-1-butoxy-4-(trans-4-pentylcyclohexyl)-cyclohexan

**Beispiel 4**

Eine Lösung von 22 g p-(2-Methyl-4-n-propylcyclohexyl)-benzoesäureamid [erhältlich aus dem Säurechlorid mit $NH_3$; die entsprechende Säure ist durch Umsetzung von Phenylmagnesiumbromid mit 2-Methyl-4-n-propylcyclohexanon (Beispiel 1), Wasserabspaltung, Hydrieren der entstandenen Doppelbindung, Friedel-Crafts-Acylierung mit Acetylchlorid und nachfolgender Haloform-Reaktion erhältlich] in 500 ml DMF wird bei 50° unter Rühren tropfenweise mit 65 g $POCl_3$ versetzt. Nach weiterem einstündigen Rühren gießt man auf Eis, arbeitet wie üblich auf und erhält p-(2-Methyl-4-n-propylcyclohexyl)-benzonitril.

Analog werden hergestellt:

p-(2-Methyl-4-ethylcyclohexyl)-benzonitril
p-(2-Methyl-4-butylcyclohexyl)-benzonitril
p-(2-Methyl-4-pentylcyclohexyl)-benzonitril
p-(2-Methyl-4-hexylcyclohexyl)-benzonitril
p-(2-Methyl-4-heptylcyclohexyl)-benzonitril
p-(2-Methyl-4-octylcyclohexyl)-benzonitril

p-(3-Methyl-4-ethylcyclohexyl)-benzonitril
p-(3-Methyl-4-propylcyclohexyl)-benzonitril
p-(3-Methyl-4-butylcyclohexyl)-benzonitril
p-(3-Methyl-4-pentylcyclohexyl)-benzonitril
p-(3-Methyl-4-hexylcyclohexyl)-benzonitril
p-(3-Methyl-4-heptylcyclohexyl)-benzonitril
p-(3-Methyl-4-octylcyclohexyl)-benzonitril

**Beispiel 6**

Man kocht 20 g p-(2-Methyl-4-n-propylcyclohexyl)-benzoesäure (Beispiel 5) 1 Std. mit 24 g $SOCl_2$, dampft ein, löst das erhaltene rohe Säurechlorid in 150 ml Toluol, versetzt mit 8 ml Pyridin und 13,2 g p-Ethylphenol und kocht 2 Stunden. Nach Abkühlen und üblicher Aufarbeitung erhält man p-(2-Methyl-4-n-propylcyclohexyl)-benzoesäure(p-ethylphenylester).

Analog werden festgestellt:

p-(2-Methyl-4-propylcyclohexyl)-benzoesäure-(p-propyl-phenylester)
p-(2-Methyl-4-propylcyclohexyl)-benzoesäure-(p-butyl-phenylester)
p-(2-Methyl-4-propylcyclohexyl)-benzoesäure-(p-pentyl-phenylester)
p-(2-Methyl-4-propylcyclohexyl)-benzoesäure-(p-methoxy-phenylester)
p-(2-Methyl-4-propylcyclohexyl)-benzoesäure-(p-ethoxy-phenylester)
p-(2-Methyl-4-propylcyclohexyl)-benzoesäure-(p-propoxy-phenylester)
p-(2-Methyl-4-propylcyclohexyl)-benzoesäure-(p-butoxy-phenylester)
p-(2-Methyl-4-propylcyclohexyl)-benzoesäure-(p-cyan-phenylester)

p-(2-Methyl-4-butylcyclohexyl)-benzoesäure-(p-propyl-phenylester)
p-(2-Methyl-4-butylcyclohexyl)-benzoesäure-(p-butyl-phenylester)
p-(2-Methyl-4-butylcyclohexyl)-benzoesäure-(p-pentyl-phenylester)
p-(2-Methyl-4-butylcyclohexyl)-benzoesäure-(p-methoxy-phenylester)
p-(2-Methyl-4-butylcyclohexyl)-benzoesäure-(p-ethoxy-phenylester)
p-(2-Methyl-4-butylcyclohexyl)-benzoesäure-(p-propoxy-phenylester)
p-(2-Methyl-4-butylcyclohexyl)-benzoesäure-(p-butoxy-phenylester)
p-(2-Methyl-4-butylcyclohexyl)-benzoesäure-(p-cyan-phenylester)

p-(2-Methyl-4-pentylcyclohexyl)-benzoesäure-(p-propyl-phenylester)
p-(2-Methyl-4-pentylcyclohexyl)-benzoesäure-(p-butyl-phenylester)
p-(2-Methyl-4-pentylcyclohexyl)-benzoesäure-(p-pentyl-phenylester)
p-(2-Methyl-4-pentylcyclohexyl)-benzoesäure-(p-methoxy-phenylester)
p-(2-Methyl-4-pentylcyclohexyl)-benzoesäure-(p-ethoxy-phenylester)
p-(2-Methyl-4-pentylcyclohexyl)-benzoesäure-(p-propoxy-phenylester)
p-(2-Methyl-4-pentylcyclohexyl)-benzoesäure-(p-butoxy-phenylester)
p-(2-Methyl-4-pentylcyclohexyl)-benzoesäure-(p-cyan-phenylester)

p-(2-Methyl-4-pentylcyclohexyl)-benzoesäure-(trans-4-ethylcyclohexylester)
p-(2-Methyl-4-pentylcyclohexyl)-benzoesäure-(trans-4-propylcyclohexylester)
p-(2-Methyl-4-pentylcyclohexyl)-benzoesäure-(trans-4-butylcyclohexylester)
p-(2-Methyl-4-pentylcyclohexyl)-benzoesäure-(trans-4-pentylcyclohexylester)

p-(2-Methyl-4-butylcyclohexyl)-benzoesäure-(trans-4-ethylcyclohexylester)
p-(2-Methyl-4-butylcyclohexyl)-benzoesäure-(trans-4-propylcyclohexylester)
p-(2-Methyl-4-butylcyclohexyl)-benzoesäure-(trans-4-butylcyclohexylester)
p-(2-Methyl-4-butylcyclohexyl)-benzoesäure-(trans-4-pentylcyclohexylester)

p-(2-Methyl-4-propylcyclohexyl)-benzoesäure-(trans-4-ethylcyclohexylester)
p-(2-Methyl-4-propylcyclohexyl)-benzoesäure-(trans-4-propylcyclohexylester)
p-(2-Methyl-4-propylcyclohexyl)-benzoesäure-(trans-4-butylcyclohexylester)
p-(2-Methyl-4-propylcyclohexyl)-benzoesäure-(trans-4-pentylcyclohexylester)

**Beispiel 7**

177,5 g trans-4-(4-n-Propyl-2-methylcyclohexyl)-cyclohexancarbonsäureamid [erhältlich durch Standardverfahren aus der entsprechenden Carbonsäure, die man wiederum durch Haloformabbau aus Beispiel 11 und anschließende Hydrierung gewinnt] wird in 150 ml $CH_2Cl_2$ suspendiert, 1 ml DMF zugesetzt. Dazu tropft man in der Siedehitze 7,3 ml Thionylchlorid und rührt 4 Stunden. Nach dem Abkühlen auf Raumtemperatur wird das Reaktionsgemisch auf Eiswasser gegeben und wie üblich aufgearbeitet. Man erhält trans-4-(trans-4-n-Propyl-2-methyl-cyclohexyl)-cyclohexancarbonitril.

Analog werden hergestellt:

trans-4-(trans-4-Ethyl-2-methylcyclohexyl)-cyclohexan-carbonitril
trans-4-(trans-4-Butyl-2-methylcyclohexyl)-cyclohexan-carbonitril
trans-4-(trans-4-Pentyl-2-methylcyclohexyl)-cyclohexan-carbonitril
trans-4-(trans-4-Heptyl-2-methylcyclohexyl)-cyclohexan-carbonitril

**Beispiel 8**

Ein Gemisch von 26,6 g trans-4-(4-n-Propyl-2-methylcyclohexyl)-cyclohexancarbonsäure und 14,2 g trans-4-n-Propylcyclohexanol werden zusammen mit einer katalytischen Menge p-Toluolsulfonsäure 2 Stunden mit Toluol am Wasserabscheider zum Sieden erhitzt. Nach dem Abkühlen wird die Toluollösung mit $Na_2CO_3$-Lösung gewaschen und wie üblich aufgearbeitet. Man erhält trans-4-(trans-4-n-Propyl-2-methyl-cyclohexyl)-cyclohexancarbonsäure-trans-4-n-propylcyclohexylester.

Analog werden hergestellt:

trans-4-(trans-4-Propyl-2-methylcyclohexyl)-cyclohexan-carbonsäure-trans-4-ethylcyclohexylester
trans-4-(trans-4-Propyl-2-methylcyclohexyl)-cyclohexan-carbonsäure-trans-4-butylcyclohexylester
trans-4-(trans-4-Propyl-2-methylcyclohexyl)-cyclohexan-carbonsäure-trans-4-pentylcyclohexylester

trans-4-(trans-4-Ethyl-2-methylcyclohexyl)-cyclohexan-carbonsäure-trans-4-ethylcyclohexylester

trans-4-(trans-4-Ethyl-2-methylcyclohexyl)-cyclohexan-carbonsäure-trans-4-propylcyclohexylester
trans-4-(trans-4-Ethyl-2-methylcyclohexyl)-cyclohexan-carbonsäure-trans-4-butylcyclohexylester
trans-4-(trans-4-Ethyl-2-methylcyclohexyl)-cyclohexan-carbonsäure-trans-4-pentylcyclohexylester

trans-4-(trans-4-Butyl-2-methylcyclohexyl)-cyclohexan-carbonsäure-trans-4-ethylcyclohexylester
trans-4-(trans-4-Butyl-2-methylcyclohexyl)-cyclohexan-carbonsäure-trans-4-propylcyclohexylester
trans-4-(trans-4-Butyl-2-methylcyclohexyl)-cyclohexan-carbonsäure-trans-4-butylcyclohexylester
trans-4-(trans-4-Butyl-2-methylcyclohexyl)-cyclohexan-carbonsäure-trans-4-pentylcyclohexylester

trans-4-(trans-4-Pentyl-2-methylcyclohexyl)-cyclohexan-carbonsäure-trans-4-ethylcyclohexylester
trans-4-(trans-4-Pentyl-2-methylcyclohexyl)-cyclohexan-carbonsäure-trans-4-propylcyclohexylester
trans-4-(trans-4-Pentyl-2-methylcyclohexyl)-cyclohexan-carbonsäure-trans-4-butylcyclohexylester
trans-4-(trans-4-Pentyl-2-methylcyclohexyl)-cyclohexan-carbonsäure-trans-4-pentylcyclohexylester

trans-4-(trans-4-Heptyl-2-methylcyclohexyl)-cyclohexan-carbonsäure-trans-4-ethylcyclohexylester
trans-4-(trans-4-Heptyl-2-methylcyclohexyl)-cyclohexan-carbonsäure-trans-4-propylcyclohexylester
trans-4-(trans-4-Heptyl-2-methylcyclohexyl)-cyclohexan-carbonsäure-trans-4-butylcyclohexylester
trans-4-(trans-4-Heptyl-2-methylcyclohexyl)-cyclohexan-carbonsäure-trans-4-pentylcyclohexylester

**Beispiel 9**

Zu einer Grignard-Lösung aus 10,7 g Mg-Spänen und 66,4 g 1-Brompentan in 500 ml Ether gibt man unter $N_2$-Atmosphäre eine Lösung von 108,7 g trans-4-(4-Propyl-2-methylcyclohexyl)-cyclohexancarbonitril (Bsp. 7) in 600 ml Toluol. Der Ether wird aus dem Reaktionsgemisch abdestilliert und dann das Reaktionsgemisch 8 Stunden am Rückfluß gekocht. Nach dem Abkühlen wird mit halbkonzentrierter HCl hydrolysiert und wie üblich aufgearbeitet. Man erhält trans-4-(trans-4-n-Propyl-2-methylcyclohexyl)-cyclohexyl-n-pentyl-keton.

Analog werden hergestellt:

4-(4-Propyl-2-methylcyclohexyl)-cyclohexyl-methyl-keton
4-(4-Propyl-2-methylcyclohexyl)-cyclohexyl-ethyl-keton
4-(4-Propyl-2-methylcyclohexyl)-cyclohexyl-propyl-keton
4-(4-Propyl-2-methylcyclohexyl)-cyclohexyl-butyl-keton

4-(4-Ethyl-2-methylcyclohexyl)-cyclohexyl-methyl-keton
4-(4-Ethyl-2-methylcyclohexyl)-cyclohexyl-ethyl-keton
4-(4-Ethyl-2-methylcyclohexyl)-cyclohexyl-propyl-keton
4-(4-Ethyl-2-methylcyclohexyl)-cyclohexyl-butyl-keton

4-(4-Butyl-2-methylcyclohexyl)-cyclohexyl-methyl-keton
4-(4-Butyl-2-methylcyclohexyl)-cyclohexyl-ethyl-keton
4-(4-Butyl-2-methylcyclohexyl)-cyclohexyl-propyl-keton
4-(4-Butyl-2-methylcyclohexyl)-cyclohexyl-butyl-keton

4-(4-Pentyl-2-methylcyclohexyl)-cyclohexyl-methyl-keton
4-(4-Pentyl-2-methylcyclohexyl)-cyclohexyl-ethyl-keton
4-(4-Pentyl-2-methylcyclohexyl)-cyclohexyl-propyl-keton
4-(4-Pentyl-2-methylcyclohexyl)-cyclohexyl-butyl-keton

**Beispiel 10**

Ein Gemisch von 32 g Keton (Beispiel 9), 30 ml Hydrazinhydrat, 53 g KOH und 300 ml Diglykol wird unter Rühren zum Sieden erhitzt und die flüchtigen Anteile aus dem Reaktionsgemisch so lange abdestilliert bis im Kolben eine Temperatur von etwa 200°C erreicht wird und sich das Hydrazon zersetzt hat. Anschließend wird das Reaktionsgemisch auf Wasser gegeben und wie üblich aufgearbeitet. Man erhält trans-4-(trans-4-n-Propyl-2-methylcyclohexyl)-1-n-hexyl-cyclohexan.

Analog werden hergestellt:

4-(4-Propyl-2-methylcyclohexyl)-1-ethylcyclohexan

4-(4-Propyl-2-methylcyclohexyl)-1-propylcyclohexan
4-(4-Propyl-2-methylcyclohexyl)-1-butylcyclohexan
4-(4-Propyl-2-methylcyclohexyl)-1-pentylcyclohexan

4-(4-Butyl-2-methylcyclohexyl)-1-ethylcyclohexan
4-(4-Butyl-2-methylcyclohexyl)-1-propylcyclohexan
4-(4-Butyl-2-methylcyclohexyl)-1-butylcyclohexan
4-(4-Butyl-2-methylcyclohexyl)-1-pentylcyclohexan

4-(4-Pentyl-2-methylcyclohexyl)-1-ethylcyclohexan
4-(4-Pentyl-2-methylcyclohexyl)-1-propylcyclohexan
4-(4-Pentyl-2-methylcyclohexyl)-1-butylcyclohexan
4-(4-Pentyl-2-methylcyclohexyl)-1-pentylcyclohexan

**Beispiel 11**

Unter Wasserausschluß und Eiskühlung tropft man zu einer kräftig gerührten Suspension von 160 g $AlCl_3$ in 400 ml 1,2-Dichlorethan 82 g Acetylchlorid und dann bei 20°C 214 g 4-Propyl-2-methylcyclohexyl-benzol (erhältlich aus 3-Propyl-2-methylcyclohexanon und Brombenzol durch Grignard-Reaktion, anschließende Wasserabspaltung und Hydrierung). Dann wird noch 1 Stunde gerührt und über Nacht stehengelassen. Zur Zerlegung des Keton-Aluminiumchlorid-Komplexes gießt man das Reaktionsgemisch auf etwa 500 ml Eis und gibt dann konzentrierte Salzsäure zu. Die organische Schicht wird abgetrennt und wie üblich aufgearbeitet. Man erhält 4-(4-Propyl-2-methylcyclohexyl)-acetophenon.

Analog werden hergestellt:

4-(4-Ethyl-2-methylcylcohexyl)-acetophenon
4-(4-Butyl-2-methylcylcohexyl)-acetophenon
4-(4-Pentyl-2-methylcylcohexyl)-acetophenon
4-(4-Heptyl-2-methylcylcohexyl)-acetophenon

**Beispiel 12**

Ein Gemisch von 23,2 g 4-(4-Propyl-2-methylcyclohexyl)-phenol [erhältlich aus 4-Propyl-2-methylcyclohexylbenzol (erhältlich durch Grignard-Reaktion von Brombenzol mit 4-n-Propyl-2-methylcyclohexanon, anschließende Wasserabspaltung und Hydrierung der Doppelbindung) durch Nitrierung, Reduktion der Nitrogruppe zum Amin und Diazotierung mit anschließender Phenolverkochung], 6,9 g $K_2CO_3$, 19 g Butyljodid und 200 ml DMF wird unter Rühren 16 Stunden auf 80°C erhitzt. Dann wird abgekühlt und wie üblich aufgearbeitet. Man erhält r-1-p-Butoxyphenyl-2-methyl-trans-4-n-propylcyclohexan.

Analog werden hergestellt:

1-p-Methoxyphenyl-2-methyl-4-propylcyclohexan
1-p-Ethoxyphenyl-2-methyl-4-propylcyclohexan
1-p-Propoxyphenyl-2-methyl-4-propylcyclohexan

1-p-Methoxyphenyl-2-methyl-4-ethylcyclohexan
1-p-Ethoxyphenyl-2-methyl-4-ethylcyclohexan
1-p-Propoxyphenyl-2-methyl-4-ethylcyclohexan
1-p-Butoxyphenyl-2-methyl-4-ethylcyclohexan

1-p-Methoxyphenyl-2-methyl-4-butylcyclohexan
1-p-Ethoxyphenyl-2-methyl-4-butylcyclohexan
1-p-Propoxyphenyl-2-methyl-4-butylcyclohexan
1-p-Butoxyphenyl-2-methyl-4-butylcyclohexan

1-p-Methoxyphenyl-2-methyl-4-pentylcyclohexan
1-p-Ethoxyphenyl-2-methyl-4-pentylcyclohexan
1-p-Propoxyphenyl-2-methyl-4-pentylcyclohexan
1-p-Butoxyphenyl-2-methyl-4-pentylcyclohexan

**Beispiel 13**

Ein Gemisch aus 23,8 g 4-trans-(4-n-Propyl-2-methylcyclohexyl)-cyclohexanol (hergestellt aus dem Phenol aus Beispiel 12 durch Hydrierung), 2,4 g NaH und 150 ml Dimethoxyethan wird unter $N_2$-Atmosphäre auf etwa 60°C erhitzt. Dann wird unter kräftigem Rühren 14 g Dimethylsulfat zugetropft. Man rührt 12 Stunden bei 60°C und arbeitet dann wie üblich auf. Man erhält trans-4-(trans-4-n-Propyl-2-methyl-cyclohexyl)-1-methoxycyclohexan.

Analog werden hergestellt:

4-(4-Propyl-2-methylcyclohexyl)-1-ethoxycyclohexan
4-(4-Propyl-2-methylcyclohexyl)-1-propoxycyclohexan
4-(4-Propyl-2-methylcyclohexyl)-1-butoxycyclohexan

4-(4-Butyl-2-methylcyclohexyl)-1-methoxycyclohexan
4-(4-Butyl-2-methylcyclohexyl)-1-ethoxycyclohexan
4-(4-Butyl-2-methylcyclohexyl)-1-propoxycyclohexan
4-(4-Butyl-2-methylcyclohexyl)-1-butoxycyclohexan

4-(4-Pentyl-2-methylcyclohexyl)-1-methoxycyclohexan
4-(4-Pentyl-2-methylcyclohexyl)-1-ethoxycyclohexan
4-(4-Pentyl-2-methylcyclohexyl)-1-propoxycyclohexan
4-(4-Pentyl-2-methylcyclohexyl)-1-butoxycyclohexan

**Beispiel 14**

Eine Lösung von 72 g 1-(p-Pentylphenyl)-4-(trans-4-n-propyl-2-methylcyclohexyl)-cyclohexen-1 (erhältlich aus 4-trans-(4-trans-n-Propyl-2-methylcyclohexylcyclohexanol durch Oxidation zum Keton und Grignard-Reaktion mit 4-Pentylbrombenzol und anschließende Wasserabspaltung) in 500 ml THF wird an 8 g 10 %-igem Pd/C bei 40°C und 1 bar bis zur Aufnahme von 0,2 mol $H_2$ hydriert. Man filtriert, dampft ein und trennt das erhaltene Isomerengemisch durch Chromatographie und Kristallisation. Man erhält trans-1-p-n-Pentylphenyl-3-(trans-4-n-propyl-2-methylcyclohexyl)-cyclohexan.

Analog werden hergestellt:

1-p-Butylphenyl-4-(4-propyl-2-methylcyclohexyl)-cyclohexan
1-p-Propylphenyl-4-(4-propyl-2-methylcyclohexyl)-cyclohexan
1-p-Ethylphenyl-4-(4-propyl-2-methylcyclohexyl)-cyclohexan
1-p-Methoxyphenyl-4-(4-propyl-2-methylcyclohexyl)-cyclohexan
1-p-Ethoxyphenyl-4-(4-propyl-2-methylcyclohexyl)-cyclohexan
1-p-Propoxyphenyl-4-(4-propyl-2-methylcyclohexyl)-cyclohexan
1-p-Butoxyphenyl-4-(4-propyl-2-methylcyclohexyl)-cyclohexan
1-p-Cyanphenyl-4-(4-propyl-2-methylcyclohexyl)-cyclohexan

1-p-Butylphenyl-4-(4-ethyl-2-methylcyclohexyl)-cyclohexan
1-p-Propylphenyl-4-(4-ethyl-2-methylcyclohexyl)-cyclohexan
1-p-Ethylphenyl-4-(4-ethyl-2-methylcyclohexyl)-cyclohexan
1-p-Methoxyphenyl-4-(4-ethyl-2-methylcyclohexyl)-cyclchexan
1-p-Ethoxyphenyl-4-(4-ethyl-2-methylcyclohexyl)-cyclohexan
1-p-Propoxyphenyl-4-(4-ethyl-2-methylcyclohexyl)-cyclohexan
1-p-Butoxyphenyl-4-(4-ethyl-2-methylcyclohexyl)-cyclohexan
1-p-Cyanphenyl-4-(4-ethyl-2-methylcyclohexyl)-cyclohexan

1-p-Butylphenyl-4-(4-butyl-2-methylcyclohexyl)-cyclohexan
1-p-Propylphenyl-4-(4-butyl-2-methylcyclohexyl)-cyclohexan
1-p-Ethylphenyl-4-(4-butyl-2-methylcyclohexyl)-cyclohexan
1-p-Methoxyphenyl-4-(4-butyl-2-methylcyclohexyl)-cyclohexan
1-p-Ethoxyphenyl-4-(4-butyl-2-methylcyclohexyl)-cyclohexan
1-p-Propoxyphenyl-4-(4-butyl-2-methylcyclohexyl)-cyclohexan
1-p-Butoxyphenyl-4-(4-butyl-2-methylcyclohexyl)-cyclohexan
1-p-Cyanphenyl-4-(4-butyl-2-methylcyclohexyl)-cyclohexan

1-p-Butylphenyl-4-(4-pentyl-2-methylcyclohexyl)-cyclohexan
1-p-Propylphenyl-4-(4-pentyl-2-methylcyclohexyl)-cyclohexan
1-p-Ethylphenyl-4-(4-pentyl-2-methylcyclohexyl)-cyclohexan

1-p-Methoxyphenyl-4-(4-pentyl-2-methylcyclohexyl)-cyclohexan
1-p-Ethoxyphenyl-4-(4-pentyl-2-methylcyclohexyl)-cyclohexan
1-p-Propoxyphenyl-4-(4-pentyl-2-methylcyclohexyl)-cyclohexan
1-p-Butoxyphenyl-4-(4-pentyl-2-methylcyclohexyl)-cyclohexan
1-p-Cyanphenyl-4-(4-pentyl-2-methylcyclohexyl)-cyclohexan

**Beispiel 15**

Eine Mischung von 43 g 4-Pentyl-4'-(trans-4-n-propyl-2-methylcyclohexylacetyl)-biphenyl [erhältlich aus trans-4-n-Propyl-2-methylcyclohexylessigsäurechlorid durch Friedel-Crafts-Acylierung von 4-Pentylbiphenyl. trans-4-n-Propyl-2-methylcyclohexylessigsäure erhält man aus 4-Propyl-2-methylbenzoesäure durch katalytische Hydrierung zur 4-Propyl-2-methylcyclohexancarbonsäure. Die anschließende Isomerisierung durch Kochen mit Thionylchlorid und die Reduktion mit $LiAlH_4$ liefert trans-4-n-Propyl-2-methylcyclohexylmethanol, dessen Tosylat mit NaCN zum trans-4-n-Propyl-2-methylcyclohexylacetonitril umgesetzt wird. Die Verseifung des Nitrils liefert die gewünschte Carbonsäure, 20 ml 80 %-iges Hydrazinhydrat und 20 g KOH wird in 240 ml Diethylenglykol 2 Stunden unter Rückfluß gekocht. Anschließend wird der Rückflußkühler durch einen Destillationsaufsatz ersetzt, die Temperatur langsam auf 195 - 200°C gesteigert und 6 Stunden bei dieser Temperatur gehalten. Es destilliert ein Gemisch von Hydrazin und Wasser über. Nach Abkühlen der Mischung verdünnt man mit 300 ml Wasser, säuert an und extrahiert mit Toluol. Die organische Phase wird mit Wasser mehrmals gewaschen und dann das Lösungsmittel im Vakuum entfernt. Der Rückstand wird durch Chromatographie und Kristallisation gereinigt. Man erhält 1-(trans-4-n-Propyl-2-methylcyclohexyl)-2-(4'-n-pentylbiphenyl-4-yl)-ethan.

Analog werden hergestellt:

1-(4-Propyl-2-methylcyclohexyl)-2-(4'-butylbiphenyl-4-yl)-ethan
1-(4-Propyl-2-methylcyclohexyl)-2-(4'-propylbiphenyl-4-yl)-ethan
1-(4-Propyl-2-methylcyclohexyl)-2-(4'-ethylbiphenyl-4-yl)-ethan
1-(4-Propyl-2-methylcyclohexyl)-2-(4'-methoxybiphenyl-4-yl)-ethan
1-(4-Propyl-2-methylcyclohexyl)-2-(4'-ethoxybiphenyl-4-yl)-ethan
1-(4-Propyl-2-methylcyclohexyl)-2-(4'-butoxybiphenyl-4-yl)-ethan
1-(4-Propyl-2-methylcyclohexyl)-2-(4'-cyanbiphenyl-4-yl)-ethan
1-(4-Ethyl-2-methylcyclohexyl)-2-(4'-butylbiphenyl-4-yl)-ethan
1-(4-Ethyl-2-methylcyclohexyl)-2-(4'-propylbiphenyl-4-yl)-ethan
1-(4-Ethyl-2-methylcyclohexyl)-2-(4'-ethylbiphenyl-4-yl)-ethan
1-(4-Ethyl-2-methylcyclohexyl)-2-(4'-methoxybiphenyl-4-yl)-ethan
1-(4-Ethyl-2-methylcyclohexyl)-2-(4'-ethoxybiphenyl-4-yl)-ethan
1-(4-Ethyl-2-methylcyclohexyl)-2-(4'-butoxybiphenyl-4-yl)-ethan
1-(4-Ethyl-2-methylcyclohexyl)-2-(4'-cyanbiphenyl-4-yl)-ethan

1-(4-Butyl-2-methylcyclohexyl)-2-(4'-butylbiphenyl-4-yl)-ethan
1-(4-Butyl-2-methylcyclohexyl)-2-(4'-propylbiphenyl-4-yl)-ethan
1-(4-Butyl-2-methylcyclohexyl)-2-(4'-ethylbiphenyl-4-yl)-ethan
1-(4-Butyl-2-methylcyclohexyl)-2-(4'-methoxybiphenyl-4-yl)-ethan
1-(4-Butyl-2-methylcyclohexyl)-2-(4'-ethoxybiphenyl-4-yl)-ethan
1-(4-Butyl-2-methylcyclohexyl)-2-(4'-butoxybiphenyl-4-yl)-ethan
1-(4-Butyl-2-methylcyclohexyl)-2-(4'-cyanbiphenyl-4-yl)-ethan

1-(4-Pentyl-2-methylcyclohexyl)-2-(4'-butylbiphenyl-4-yl)-ethan
1-(4-Pentyl-2-methylcyclohexyl)-2-(4'-propylbiphenyl-4-yl)-ethan
1-(4-Pentyl-2-methylcyclohexyl)-2-(4'-ethylbiphenyl-4-yl)-ethan
1-(4-Pentyl-2-methylcyclohexyl)-2-(4'-methoxybiphenyl-4-yl)-ethan
1-(4-Pentyl-2-methylcyclohexyl)-2-(4'-ethoxybiphenyl-4-yl)-ethan
1-(4-Pentyl-2-methylcyclohexyl)-2-(4'-butoxybiphenyl-4-yl)-ethan
1-(4-Pentyl-2-methylcyclohexyl)-2-(4'-cyanbiphenyl-4-yl)-ethan

**Beispiel 16**

Eine Lösung von 41 g 4-Pentyl-2-fluor-4'-(trans-4-n-propyl-2-methylcyclohexylacetyl)-biphenyl [erhältlich aus trans-4-Propyl-2-methylessigsäurechlorid durch Friedel-Crafts-Acylierung von 2-Fluor-4-Pentyl-biphenyl] in 200 ml THF wird bei 50°C unter 3 bar Druck in Gegenwart von 12 g Pd/C hydriert. Nachdem 0,2 mol $H_2$ aufgenommen worden sind, wird die Hydrierung abgebrochen, das Reaktionsgemisch filtriert und dann das

Lösungsmittel im Vakuum abgezogen. Der Rückstand wird durch Chromatographie und Kristallisation gereinigt. Man erhält 1-(trans-4-n-Propyl-2-methylcyclohexyl)-2-(4'-n-pentyl-2'-fluorbiphenyl-4-yl)-ethan.

Analog werden hergestellt:

1-(4-Propyl-2-methylcyclohexyl)-2-(4'-butyl-2'-fluorbiphenyl-4-yl)-ethan
1-(4-Propyl-2-methylcyclohexyl)-2-(4'-propyl-2'-fluorbiphenyl-4-yl)-ethan
1-(4-Propyl-2-methylcyclohexyl)-2-(4'-ethyl-2'-fluorbiphenyl-4-yl)-ethan
1-(4-Propyl-2-methylcyclohexyl)-2-(4'-methyl-2'-fluorbiphenyl-4-yl)-ethan

**Beispiel 17**

Ein Gemisch von 28,4 g 1-(p-Pentylphenyl)-2-oxo-trans-4-n-propylcyclohexan [erhältlich aus 1-(p-n-Pentylphenyl)-4-propylcyclohexen durch Hydroborierung, Spaltung der Borverbindung mit alkalischem $H_2O_2$ und anschließende Oxidation des Alkohols mit Pyridiniumchlorochromat zum Keton] und 13,3 g DAST (Diethylaminoschwefeltrifluorid) wird langsam auf 80° C erwärmt und bei dieser Temperatur 1 Stunde gerührt. Nach dem Abkühlen wird auf Eiswasser geschüttet und dann mit Methylenchlorid extrahiert. Nach dem Trocknen der organischen Phase wird das Lösungsmittel abgezogen und der Rückstand durch Chromatographie gereinigt. Man erhält r-1-(p-n-Pentylphenyl)-2,2-difluor-trans-4-n-propylcyclohexan.

Analog werden hergestellt:

1-(p-Butylphenyl)-2,2-difluor-4-propylcyclohexan
1-(p-Propylphenyl)-2,2-difluor-4-propylcyclohexan
1-(p-Ethylphenyl)-2,2-difluor-4-propylcyclohexan
1-(p-Methoxyphenyl)-2,2-difluor-4-propylcyclohexan
1-(p-Ethoxyphenyl)-2,2-difluor-4-propylcyclohexan
1-(p-Propoxyphenyl)-2,2-difluor-4-propylcyclohexan
1-(p-Cyanphenyl)-2,2-difluor-4-propylcyclohexan

1-(p-Butylphenyl)-2,2-difluor-4-butylcyclohexan
1-(p-Propylphenyl)-2,2-difluor-4-butylcyclohexan
1-(p-Ethylphenyl)-2,2-difluor-4-butylcyclohexan
1-(p-Methoxyphenyl)-2,2-difluor-4-butylcyclohexan
1-(p-Ethoxyphenyl)-2,2-difluor-4-butylcyclohexan
1-(p-Propoxyphenyl)-2,2-difluor-4-butylcyclohexan
1-(p-Cyanphenyl)-2,2-difluor-4-butylcyclohexan

1-(p-Butylphenyl)-2,2-difluor-4-pentylcyclohexan
1-(p-Propylphenyl)-2,2-difluor-4-pentylcyclohexan
1-(p-Ethylphenyl)-2,2-difluor-4-pentylcyclohexan
1-(p-Methoxyphenyl)-2,2-difluor-4-pentylcyclohexan
1-(p-Ethoxyphenyl)-2,2-difluor-4-pentylcyclohexan
1-(p-Propoxyphenyl)-2,2-difluor-4-pentylcyclohexan
1-(p-Cyanphenyl)-2,2-difluor-4-pentylcyclohexan

**Beispiel 18**

Ein Gemisch aus 28,8 g trans-4-n-Propyl-2-hydroxy-1-(p-pentylphenyl)cyclohexan [erhältlich aus dem entsprechenden Keton durch Reduktion mit $LiAlH_4$] und 2,4 g NaH in 150 ml Dimethoxyethan wird unter $N_2$-Atmosphäre auf 60° C erhitzt. Dann gibt man unter kräftigem Rühren langsam 13 g Dimethylsulfat zu und rührt weitere 12 Stunden. Das Reaktionsgemisch wird wie üblich aufgearbeitet und das Produkt durch Chromatographie gereinigt. Man erhält r-1-(p-n-Pentylphenyl)-2-methoxy-trans-4-n-propyl-cyclohexan.

Analog werden hergestellt:

1-(p-Butylphenyl)-2-methoxy-4-propylcyclohexan
1-(p-Propylphenyl)-2-methoxy-4-propylcyclohexan
1-(p-Ethylphenyl)-2-methoxy-4-propylcyclohexan
1-(p-Methoxyphenyl)-2-methoxy-4-propylcyclohexan
1-(p-Ethoxyphenyl)-2-methoxy-4-propylcyclohexan
1-(p-Propoxyphenyl)-2-methoxy-4-propylcyclohexan
1-(p-Cyanphenyl)-2-methoxy-4-propylcyclohexan

1-(p-Butylphenyl)-2-methoxy-4-butylcyclohexan
1-(p-Propylphenyl)-2-methoxy-4-butylcyclohexan
1-(p-Ethylphenyl)-2-methoxy-4-butylcyclohexan
1-(p-Methoxyphenyl)-2-methoxy-4-butylcyclohexan
1-(p-Ethoxyphenyl)-2-methoxy-4-butylcyclohexan
1-(p-Propoxyphenyl)-2-methoxy-4-butylcyclohexan
1-(p-Cyanphenyl)-2-methoxy-4-butylcyclohexan

1-(p-Butylphenyl)-2-methoxy-4-pentylcyclohexan
1-(p-Propylphenyl)-2-methoxy-4-pentylcyclohexan
1-(p-Ethylphenyl)-2-methoxy-4-pentylcyclohexan
1-(p-Methoxyphenyl)-2-methoxy-4-pentylcyclohexan
1-(p-Ethoxyphenyl)-2-methoxy-4-pentylcyclohexan
1-(p-Propoxyphenyl)-2-methoxy-4-pentylcyclohexan
1-(p-Cyanphenyl)-2-methoxy-4-pentylcyclohexan

**Beispiel 19**

Eine Lösung von 24 g 2-(p-Ethylphenyl)-5-n-propylcyclohexancarbonsäureamid in 500 ml DMF wird bei 50° unter Rühren tropfenweise mit 65 g $POCl_3$ versetzt. Nach weiterem einstündigen Rühren gießt man auf Eis, arbeitet wie üblich auf und erhält 2-(p-Ethylphenyl)-5-n-propylcyclohexancarbonitril.

Analog werden hergestellt:

2-(p-Propylphenyl)-5-propylcyclohexancarbonitril
2-(p-Butylphenyl)-5-propylcyclohexancarbonitril
2-(p-Pentylphenyl)-5-propylcyclohexancarbonitril

2-(p-Ethylphenyl)-5-butylcyclohexancarbonitril
2-(p-Propylphenyl)-5-butylcyclohexancarbonitril
2-(p-Butylphenyl)-5-butylcyclohexancarbonitril
2-(p-Pentylphenyl)-5-butylcyclohexancarbonitril

2-(p-Ethylphenyl)-5-pentylcyclohexancarbonitril
2-(p-Propylphenyl)-5-pentylcyclohexancarbonitril
2-(p-Butylphenyl)-5-pentylcyclohexancarbonitril
2-(p-Pentylphenyl)-5-pentylcyclohexancarbonitril

**Beispiel 20**

Zu einer Lösung von 0,01 Mol r-2-Methyl-trans-4-n-propylcyclohexan-cis-1-carbonsäurechlorid [nach literaturbekannten Methoden erhältlich aus cis-3-Methyl-4-carboxycyclohexanon (beschrieben in Chem. Ber. 116 (5), 1180 (1983)] in 10 ml Pyridin gibt man 0,01 Mol p-Cyanphenol in 15 ml Pyridin, rührt 20 Stunden bei 0° und arbeitet wie üblich auf. Man erhält r-2-Methyl-trans-4-n-propyl-cyclohexan-cis-1-carbonsäure-p-cyanphenylester.

Analog werden hergestellt:

r-2-Methyl-trans-4-propylcyclohexan-cis-1-carbonsäure-p-ethylphenylester
r-2-Methyl-trans-4-propylcyclohexan-cis-1-carbonsäure-p-propylphenylester
r-2-Methyl-trans-4-propylcyclohexan-cis-1-carbonsäure-p-butylphenylester
r-2-Methyl-trans-4-propylcyclohexan-cis-1-carbonsäure-p-pentylphenylester
r-2-Methyl-trans-4-propylcyclohexan-cis-1-carbonsäure-p-methoxyphenylester
r-2-Methyl-trans-4-propylcyclohexan-cis-1-carbonsäure-p-ethoxyphenylester
r-2-Methyl-trans-4-propylcyclohexan-cis-1-carbonsäure-p-propoxyphenylester
r-2-Methyl-trans-4-propylcyclohexan-cis-1-carbonsäure-p-butoxyphenylester

r-2-Methyl-trans-4-butylcyclohexan-cis-1-carbonsäure-p-ethylphenylester
r-2-Methyl-trans-4-butylcyclohexan-cis-1-carbonsäure-p-propylphenylester
r-2-Methyl-trans-4-butylcyclohexan-cis-1-carbonsäure-p-butylphenylester
r-2-Methyl-trans-3-butylcyclohexan-cis-1-carbonsäure-p-pentylphenylester
r-2-Methyl-trans-4-butylcyclohexan-cis-1-carbonsäure-p-methoxyphenylester

r-2-Methyl-trans-4-butylcyclohexan-cis-1-carbonsäure-p-ethoxyphenylester
r-2-Methyl-trans-4-butylcyclohexan-cis-1-carbonsäure-p-propoxyphenylester
r-2-Methyl-trans-4-butylcyclohexan-cis-1-carbonsäure-p-butoxyphenylester
r-2-Methyl-trans-4-butylcyclohexan-cis-1-carbonsäure-p-cyanphenylester

r-2-Methyl-trans-4-pentylcyclohexan-cis-1-carbonsäure-p-ethylphenylester
r-2-Methyl-trans-4-pentylcyclohexan-cis-1-carbonsäure-p-propylphenylester
r-2-Methyl-trans-4-pentylcyclohexan-cis-1-carbonsäure-p-butylphenylester
r-2-Methyl-trans-4-pentylcyclohexan-cis-1-carbonsäure-p-pentylphenylester
r-2-Methyl-trans-4-pentylcyclohexan-cis-1-carbonsäure-p-methoxyphenylester
r-2-Methyl-trans-4-pentylcyclohexan-cis-1-carbonsäure-p-ethoxyphenylester
r-2-Methyl-trans-4-pentylcyclohexan-cis-1-carbonsäure-p-propoxyphenylester
r-2-Methyl-trans-4-pentylcyclohexan-cis-1-carbonsäure-p-butoxyphenylester
r-2-Methyl-trans-4-pentylcyclohexan-cis-1-carbonsäure-p-cyanphenylester

**Beispiel 21**

a) Zu einer Lösung von 36 g 3-Methylcyclohexanon in 630 ml THF gibt man 105 g 2,4,6-Triisopropylbenzolsulfonsäurehydrazid und rührt 2 Stunden bei Raumtemperatur. Hierauf werden 450 ml Methanol und 65 g KCN zugefügt und 2 Stunden am Rückfluß erhitzt. Nach üblicher Aufarbeitung und anschließender fraktionierter Destillation erhält man 3-Methylcyclohexacarbonitril.

b) Zu einer Lösung von 17 g 3-Methylcyclohexancarbonitril in 140 ml THF gibt man bei -78° eine Lithiumdiisopropylamid-Lösung (hergestellt aus 140 ml THF, 16 g Diisopropylamin und 100 ml 1,6 molarer Butyllithium-Lösung in Hexan), nach 2 Stunden 25 g Diethylcarbonat und rührt noch eine Stunde. Nach Erwarmen auf Raumtemperatur und Gießen auf 300 ml Eiswasser, wird mit verd. Salzsäure neutralisiert. Nach üblicher Aufarbeitung und Destillation erhält man 3-Methyl-1-cyan-cyclohexancarbonsäureethylester.

Dieser Ester wird durch Erwärmen mit ethanolischer Kalilauge verseift. Nach Neutralisation, Extraktion mit Methyl-tert.-butylether und Abdestillieren des Lösungsmittels erhält man 1-Cyan-3-methylcyclohexan-carbonsäure.

c) Zu einem Gemisch aus 16 g 1-Cyan-3-methyl-cyclohexancarbonsäure, 25 g 4'-Hexyl-biphenylol-4, 1,2 g 4-Diethylaminopyridin und 200 ml Methylenchlorid gibt man bei Raumtemperatur eine Lösung von 23 g Dicyclohexylcarbodiimid in 50 ml $CH_2Cl_2$ und rührt 2 Stunden. Nach üblicher Aufarbeitung und säulenchromatographischer Trennung (Kieselgel/Toluol) erhält man als Reinprodukt durch Umkristallisation das 4'-Hexylbiphenyl-4-yl-1-cyan-3-methylcyclohexancarboxylat, Fp. 51°.

Analog werden hergestellt:

4'-Ethylbiphenyl-4-yl-1-cyan-3-methylcyclohexancarboxylat
4'-Propylbiphenyl-4-yl-1-cyan-3-methylcyclohexancarboxylat
4'-Butylbiphenyl-4-yl-1-cyan-3-methylcyclohexancarboxylat
4'-Pentylbiphenyl-4-yl-1-cyan-3-methylcyclohexancarboxylat
4'-Heptylbiphenyl-4-yl-1-cyan-3-methylcyclohexancarboxylat
4'-Ethoxybiphenyl-4-yl-1-cyan-3-methylcyclohexancarboxylat
4'-Propoxybiphenyl-4-yl-1-cyan-3-methylcyclohexancarboxylat
4'-Butoxybiphenyl-4-yl-1-cyan-3-methylcyclohexancarboxylat
4'-Pentoxybiphenyl-4-yl-1-cyan-3-methylcyclohexancarboxylat
4'-Hexoxybiphenyl-4-yl-1-cyan-3-methylcyclohexancarboxylat
4'-Heptoxybiphenyl-4-yl-1-cyan-3-methylcyclohexancarboxylat

4'-Ethylbiphenyl-4-yl-1-cyan-3-ethylcyclohexancarboxylat
4'-Propylbiphenyl-4-yl-1-cyan-3-ethylcyclohexancarboxylat
4'-Butylbiphenyl-4-yl-1-cyan-3-ethylcyclohexancarboxylat
4'-Pentylbiphenyl-4-yl-1-cyan-3-ethylcyclohexancarboxylat
4'-Hexylbiphenyl-4-yl-1-cyan-3-ethylcyclohexancarboxylat
4'-Heptylbiphenyl-4-yl-1-cyan-3-ethylcyclohexancarboxylat
4'-Ethoxybiphenyl-4-yl-1-cyan-3-ethylcyclohexancarboxylat
4'-Propoxybiphenyl-4-yl-1-cyan-3-ethylcyclohexancarboxylat
4'-Butoxybiphenyl-4-yl-1-cyan-3-ethylcyclohexancarboxylat
4'-Pentoxybiphenyl-4-yl-1-cyan-3-ethylcyclohexancarboxylat
4,-Hexoxybiphenyl-4-yl-1-cyan-3-ethylcyclohexancarboxylat
4'-Heptoxybiphenyl-4-yl-1-cyan-3-ethylcyclohexancarboxylat

4'-Ethylbiphenyl-4-yl-1-cyan-3-chlorcyclohexancarboxylat
4'-Propylbiphenyl-4-yl-1-cyan-3-chlorcyclohexancarboxylat

4'-Butylbiphenyl-4-yl-1-cyan-3-chlorcyclohexancarboxylat
4'-Pentylbiphenyl-4-yl-1-cyan-3-chlorcyclohexancarboxylat
4'-Hexylbiphenyl-4-yl-1-cyan-3-chlorcyclohexancarboxylat
4'-Heptylbiphenyl-4-yl-1-cyan-3-chlorcyclohexancarboxylat
4'-Ethoxybiphenyl-4-yl-1-cyan-3-chlorcyclohexancarboxylat
4'-Propoxybiphenyl-4-yl-1-cyan-3-chlorcyclohexancarboxylat
4'-Butoxybiphenyl-4-yl-1-cyan-3-chlorcyclohexancarboxylat
4'-Pentoxybiphenyl-4-yl-1-cyan-3-chlorcyclohexancarboxylat
4'-Hexoxybiphenyl-4-yl-1-cyan-3-chlorcyclohexancarboxylat
4'-Heptoxybiphenyl-4-yl-1-cyan-3-chlorcyclohexancarboxylat

**Beispiel 22**

Zu einem Gemisch aus 3,1 g 3-Methylcyclohexancarbonitril und 30 ml THF gibt man bei -78° eine Lösung von Lithiumdiisopropylamid (hergestellt aus 3,3 g Diisopropylamin, 30 ml THF und 19 ml 1,6 molarer Butyllithium-Lösung in Hexan) und dann eine Lösung von 9,7 g 1-Brom-2-(4'-pentylbiphenyl-4-yl)-ethan in 30 ml THF. Die Aufarbeitung erfolgt analog Beispiel 1 und man erhält 1-(1-Cyan-3-methylcyclohexyl)-2-(4'-pentylbiphenyl-4-yl)ethan, Fp. 73°.
Analog werden hergestellt:

1-(1-Cyan-3-methylcyclohexyl)-2-(4'-ethylbiphenyl-4-yl)-ethan
1-(1-Cyan-3-methylcyclohexyl)-2-(4'-propylbiphenyl-4-yl)-ethan
1-(1-Cyan-3-methylcyclohexyl)-2-(4'-butylbiphenyl-4-yl)-ethan
1-(1-Cyan-3-methylcyclohexyl)-2-(4'-hexylbiphenyl-4-yl)-ethan
1-(1-Cyan-3-methylcyclohexyl)-2-(4'-heptylbiphenyl-4-yl)-ethan
1-(1-Cyan-3-methylcyclohexyl)-2-(4'-ethoxybiphenyl-4-yl)-ethan
1-(1-Cyan-3-methylcyclohexyl)-2-(4'-propoxybiphenyl-4-yl)-ethan
1-(1-Cyan-3-methylcyclohexyl)-2-(4'-butoxybiphenyl-4-yl)-ethan
1-(1-Cyan-3-methylcyclohexyl)-2-(4'-pentoxybiphenyl-4-yl)-ethan
1-(1-Cyan-3-methylcyclohexyl)-2-(4'-hexoxybiphenyl-4-yl)-ethan
1-(1-Cyan-3-methylcyclohexyl)-2-(4'-heptoxybiphenyl-4-yl)-ethan

1-(1-Cyan-3-ethylcyclohexyl)-2-(4'-ethylbiphenyl-4-yl)-ethan
1-(1-Cyan-3-ethylcyclohexyl)-2-(4'-propylbiphenyl-4-yl)-ethan
1-(1-Cyan-3-ethylcyclohexyl)-2-(4'-butylbiphenyl-4-yl)-ethan
1-(1-Cyan-3-ethylcyclohexyl)-2-(4'-pentylbiphenyl-4-yl)-ethan
1-(1-Cyan-3-ethylcyclohexyl)-2-(4'-hexylbiphenyl-4-yl)-ethan
1-(1-Cyan-3-ethylcyclohexyl)-2-(4'-heptylbiphenyl-4-yl)-ethan
1-(1-Cyan-3-ethylcyclohexyl)-2-(4'-ethoxybiphenyl-4-yl)-ethan
1-(1-Cyan-3-ethylcyclohexyl)-2-(4'-propoxybiphenyl-4-yl)-ethan
1-(1-Cyan-3-ethylcyclohexyl)-2-(4'-butoxybiphenyl-4-yl)-ethan
1-(1-Cyan-3-ethylcyclohexyl)-2-(4'-pentoxybiphenyl-4-yl)-ethan
1-(1-Cyan-3-ethylcyclohexyl)-2-(4'-hexoxybiphenyl-4-yl)-ethan
1-(1-Cyan-3-ethylcyclohexyl)-2-(4'-heptoxybiphenyl-4-yl)-ethan

**Beispiel 23**

Analog Beispiel 22 erhält man 1-(1-Cyan-3-methylcyclo-hexyl)-2-[4-(4-pentylcyclohexyl)phenyl-1-yl]-ethan, Fp. 92°, durch Umsetzung von 1-Brom-2-[4-(4-pentylcyclo-hexyl)phenyl-1-yl]-ethan mit 3-Methylcyclohexancarbonitril.
Analog werden hergestellt:

1-(1-Cyan-3-methylcyclohexyl)-2-[4-(4-ethylcyclohexyl)-phenyl-1-yl]-ethan
1-(1-Cyan-3-methylcyclohexyl)-2-[4-(4-propylcyclohexyl)-phenyl-1-yl]-ethan
1-(1-Cyan-3-methylcyclohexyl)-2-[4-(4-butylcyclohexyl)-phenyl-1-yl]-ethan
1-(1-Cyan-3-methylcyclohexyl)-2-[4-(4-hexylcyclohexyl)-phenyl-1-yl]-ethan
1-(1-Cyan-3-methylcyclohexyl)-2-[4-(4-heptylcyclohexyl)-phenyl-1-yl]-ethan.

Es folgen Beispiele für FK-Phasen mit einem Gehalt an mindestens einer Verbindung der Formel I:

**Beispiel A**

Man stellt eine flüssigkeitskristalline Phase her, bestehend aus:

17 % p-(trans-4-Propylcyclohexyl)-benzonitril,
22 % p-(trans-4-Butylcyclohexyl)-benzonitril,
24 % p-(trans-4-Pentylcyclohexyl)-benzonitril,
14 % p-(trans-4-Heptylcyclohexyl)-benzonitril,
18 % 4-Ethyl-4'-(trans-4-pentylcyclohexyl)-biphenyl und
15 % 2-Methyl-1-(p-pentylphenyl)-4-(trans-4-propylcyclohexyl)-cyclohexan

**Beispiel B**

Man stellt eine flüssigkristalline Phase her, bestehend aus:

16 % trans-1-(p-Ethoxyphenyl)-4-propylcyclohexan,
12 % trans-1-(p-Butoxyphenyl)-4-propylcyclohexan,
16 % p-(trans-4-Propylcyclohexyl)-benzonitril,
23 % p-(trans-4-Pentylcyclohexyl)-benzonitril,
15 % p-(trans-4-Heptylcyclohexyl)-benzonitril und
18 % 2-Methyl-1-(p-pentylphenyl)-4-(trans-4-propylcyclohexyl)-cyclohexan

**Patentansprüche**

1. Cyclohexanderivate der Formel I

$$R^1\text{-}A^1\text{-}Z^1\text{-}A^2\text{-}R^2 \qquad I$$

worin

| | |
|---|---|
| $R^1$ und $R^2$ | jeweils eine Alkylgruppe mit 1 - 10 C-Atomen, worin auch eine oder zwei nicht benachbarte $CH_2$-Gruppen durch O-Atome und/ oder -CO-Gruppen und/oder -CO-O-Gruppen und/oder -CH=CH-Gruppen ersetzt sein können, einer der Reste $R^1$ und $R^2$ auch H, F, Cl, Br, CN oder $R^3$-$A1^3$-$Z^2$-, |
| $A^1$ | -A-, $A^4$-A- oder -A-$A^4$-, |
| A | eine in 2-, 3-, 5- und/oder 6-Stellung ein- oder mehrfach durch F und/oder Cl und oder Br und/oder CN und/oder eine Alkylgruppe oder eine fluorierte Alkylgruppe mit jeweils 1 - 10 C-Atomen, worin auch eine oder zwei nicht benachbarte $CH_2$-Gruppen durch O-Atome und/oder -CO-Gruppen und oder -CO-O-Gruppen ersetzt sein können, substituierte trans-1,4-Cyclohexylengruppe, die gegebenenfalls auch in 1- und/oder 4-Stellung substituiert sein kann, |
| $A^2$, $A^3$ und $A^4$ | jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome und/oder NO ersetzt sein können, 1,4-Cyclohexylen, worin auch ein oder zwei nicht benachbarte $CH_2$-Gruppen durch O-Atome ersetzt sein können, 1,3-Dithian-2,5-diyl, Piperidin-1,4-diyl, 1,4-Bicyclo(2,2,2)-octylen-, Decahydronaphthalin-2,6-diyl- oder 1,2,3,4-Tetrahydronaphthalin-2,6-diyl-Gruppen, |
| $Z^1$ und $Z^2$ | jeweils -CO-O-, -O-CO-, -$CH_2CH_2$-, $OCH_2$-, -$CH_2O$ oder eine-Einfachbindung, und |
| $R^3$ | H, eine Alkylgruppe mit 1 - 10, worin auch eine oder zwei nicht benachbarte $CH_2$-Gruppen durch O-Atome und oder -CO-Gruppen und/oder -CO-O-Gruppen und/oder -CH=CH- Gruppen ersetzt sein können, F, Cl, Br oder CN bedeutet, |

mit der Maßgabe, daß im Falle $Z^1$ = -CO-O- $A^1$ in β-Position zur -CO-O-Brücke keinen äquatorialen Substituenten trägt.

2. Verfahren zur Herstellung von Cyclohexanderivaten der Formel I nach Anspruch 1, dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt, oder daß man zur Herstellung von Estern der Formel I eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Dioxanderivaten der Formel I einen entsprechenden Aldehyd mit einem entsprechenden Diol umsetzt,

oder daß man zur Herstellung von Nitrilen der Formel I ein entsprechendes Carbonsäureamid dehydratisiert oder ein entsprechendes Carbonsäurehalogenid mit Sulfamid umsetzt.

3. Verwendung der Verbindungen der Formel I nach Anspruch 1 als Komponenten flüssigkristalliner Phasen.

4. Flüssigkristallphase mit mindestens zwei flüssigkristallinen Komponenten, dadurch gekennzeichnet, daß sie mindestens eine querpolarisierte 1,4-Cyclohexylenverbindung enthält, worin mindestens eine $CH_2$-Gruppe durch eine -CHCN- oder -CH-Halogen-Gruppe ersetzt ist.

5. Flüssigkristalline Phase nach Anspruch 4, dadurch gekennzeichnet, daß mindestens eine Komponente eine Verbindung der Formel I nach Anspruch 1 ist.

6. Flüssigkristallanzeigeelement, dadurch gekenzeichnet, daß es eine Phase nach Anspruch 4 oder 5 enthält.

7. Elektrooptisches Anzeigeelement nach Anspruch 6, dadurch gekennzeichnet, daß es als Dielektrikum eine Phase nach Anspruch 4 oder 5 enthält.

**Claims**

1. Cyclohexane derivatives of the formula I

$R^1$-$A^1$-$Z^1$-$A^1$-$R^2$ I

in which

$R^1$ and $R^2$ are each an alkyl group having 1 - 10 C atoms, in which one or two non-adjacent $CH_2$ groups can also be replaced by O atoms and/or -CO- groups and/or -CO-O- groups and/or -CH=CH- groups, one of the radicals $R^1$ and $R^2$ also being H, F, Cl, Br, CN or $R^3$-$A^3$-$Z^2$-,

$A^1$ is -A-, $A^4$-A- or -A-$A^4$-,

A is a trans-1,4-cyclohexylene group which can be substituted in the 2-, 3-, 5- and/or 6-position one or more times by F and/or Cl and/or Br and/or CN and/or an alkyl group or a fluorinated alkyl group which each have 1 - 10 C atoms and in which one or two non-adjacent $CH_2$ groups can also be replaced by O atoms and/or -CO- groups and/or -CO-O- groups, and which may also be substituted in the 1- and/or 4-position,

$A^2$, $A^3$ and $A^4$ are each 1,4-phenylene which is unsubstituted or substituted by one or two F and/or Cl atoms and/or $CH_3$ groups and/or CN groups and in which one or two CH groups can also be replaced by N atoms and/or NO; 1,4-cyclohexylene in which one or two non-adjacent $CH_2$ groups can also be replaced by O atoms; or 1,3-dithiane-2,5-diyl, piperidine-1,4-diyl, 1,4-bicyclo-(2,2,2)octylene, decahydronaphthalene-2,6-diyl or 1,2,3,4-tetrahydronaphthalene-2,6-diyl groups,

$Z^1$ and $Z^2$ are each -CO-O-, -O-CO-, -$CH_2CH_2$-, -$OCH_2$-, -$CH_2O$ or a single bond, and

$R^3$ is H, an alkyl group having 1 - 10 (sic), in which one or two non-adjacent $CH_2$ groups can also be replaced by O atoms and/or -CO- groups and/or -CO-O- groups and/or CH=CH- groups, or is F, Cl, Br or CN,

with the proviso that in the case of $Z^1$ = -CO-O- $A^1$ carries no equatorial substituent in the β-position relative to the -CO-O- bridge.

2. Process for preparing cyclohexane derivatives of the formula I according to Claim 1, characterized in that a compound which otherwise conforms to the formula I but contains in place of H atoms one or more reducible groups and/or C-C bonds is treated with a reducing agent,

or in that, to prepare esters of the formula I, an appropriate carboxylic acid or one of its reactive derivatives is reacted with an appropriate alcohol or one of its reactive derivatives,

or in that, to prepare dioxane derivatives of the formula I, an appropriate aldehyde is reacted with an appropriate diol,

or in that, to prepare nitriles of the formula I, an appropriate carboxamide is dehydrated or an appropriate carbonyl halide is reacted with sulfamide.

3. Use of the compounds of the formula I according to Claim 1, as components of liquid-crystalline phases.

4. Liquid crystal phase having at least two liquid-crystalline components, characterized in that it contains at least one transversely polarized 1,4-cyclohexylene compound in which at least one $CH_2$ group is replaced by a -CHCN- or -CH-halogen- group.

5. Liquid-crystalline phase according to Claim 4, characterized in that at least one component is a compound of the formula I according to Claim 1.

6. Liquid crystal display element, characterized in that it contains a phase according to Claim 4 or 5.

7. Electro-optical display element according to Claim 6, characterized in that the dielectric it contains is a phase according to Claim 4 or 5.

**Revendications**

1. Dérivés du cyclohexane de formule I

$R^1$-$A^1$-$Z^1$-$A^2$-$R^2$ I

où

$R^1$ et $R^2$ représentent un groupe alkyle ayant 1 à 10 atomes de C, un ou deux groupes $CH_2$ non voisins pouvant être également remplacés par des atomes d'oxygène et/ou par des groupes -CO et/ou par des groupes -CO-O- et/ou par des groupes -CH=CH, un des radicaux $R^1$ et $R^2$ représentant aussi H, F, Cl, Br, CN ou $R^3$-$A^3$-$Z^2$-,

$A^1$ représente -A-, $A^4$-A- ou -A-$A^4$-,

A représente un groupe trans-1,4-cyclohexylène substitué en position 2, 3, 5 et/ou 6 une ou plusieurs fois par F et/ou Cl et/ou par Br et/ou par CN et/ou par un groupe alkyle ou par un groupe alkyle fluoré ayant 1 à 10 atomes de C, un ou deux groupes $CH_2$ non voisins pouvant être également remplacés par des atomes d'oxygène et/ou des groupes -CO et/ou des groupes -CO-O, le groupe trans-1,4-cyclohexylène pouvant être également éventuellement substitué en position 1 et/ou 4,

$A^2$, $A^3$ et $A^4$ représentent le 1,4-phénylène non substitué ou substitué par un ou deux atomes de F et/ou de Cl et/ou par des groupes $CH_3$ et/ou par des groupes CN, un ou deux groupes CH pouvant être remplacé par des atomes de N et/ou NO, le 1,4-cyclohexylène, un ou deux groupes $CH_2$ non voisins pouvant être remplacés par des atomes d'oxygène, le 1,3-dithian-2,5-diyl, des groupes pipéridine-1,4-diyl, 1,4-bicyclo(2,2,2)-octylène, décahydronaphtalène-2,6-diyl ou 1,2,3,4-tétrahydronaphtalène-2,6-diyl,

$Z^1$ et $Z^2$ représentent -CO-O-, -O-CO-, $-CH_2CH_2-$, $-OCH_2-$, $-CH_2O$ ou une liaison simple, et

$R^3$ représente H, un groupe alkyle ayant 1 à 10 atomes de C, un ou deux groupes $CH_2$ non voisins pouvant être remplacés par des atomes d'oxygène et/ou des groupes -CO et/ou des groupes -CO-O et/ou des groupes -CH=CH, F, Cl, Br ou CN,

sous réserve que, dans le cas où $Z^1$ = -CO-O-, $A^1$ porte aucun substituant équatorial en position β par rapport au pont -CO-O.

2. Procédé de préparation des dérivés du cyclohexane de formule I selon la revendication 1, caractérisé en ce que l'on traite avec un agent réducteur un composé correspondant à la formule I, mais contenant à la place d'atomes de H un ou plusieurs groupes réductibles et/ou des liaisons C-C ou en ce que l'on fait réagir, pour la préparation des esters de formule I, un acide carboxylique correspondant ou un des dérivés de ces esters réactifs avec un alcool correspondant ou avec un des dérivés de ces alcools réactifs,

ou en ce que l'on fait réagir, pour la préparation de dérivés du dioxanne de formule I, un aldéhyde correspondant avec un diol correspondant,

ou en ce que l'on déshydrate, pour la préparation des nitriles de formule I, un amide d'acide carboxylique correspondant ou en ce que l'on fait réagir un halogénure d'acide carboxylique correspondant avec un sulfamide.

3. Utilisation des composés de formule I selon la revendication 1 comme composants de phases à cristaux liquides.

4. Phase à cristaux liquides avec au moins deux composants à cristaux liquides, caractérisée en ce qu'elle contient au moins un composé 1,4-cyclohexylène polarisé transversalement, au moins un groupe $CH_2$ étant remplacé par un groupe -CHCN ou un groupe -CH halogène.

5. Phase à cristaux liquides selon la revendication 4, caractérisée en ce qu'au moins un composant est un composé de formule I selon la revendication 1.

6. Elément d'affichage à cristaux liquides, caractérisé en ce qu'il contient une phase selon la revendication 4 ou 5.

7. Elément d'affichage électronique selon la revendication 6, caractérisé en ce qu'il comprend une phase selon la revendication 4 ou 5 comme diélectrique.